# EUROPÄISCHE PATENTANMELDUNG

(11) **EP 2 695 639 A1**
(43) Veröffentlichungstag der Anmeldung: **12.02.2014**
(21) Anmeldenummer: 13176967.1
(22) Anmeldetag: 18.07.2013
(51) Int. Cl.: A61M 37/00, A61B 19/00

(54) **Verfahren und Vorrichtung zum gezielten Aufbringen eines Flüssigkeitsstrahls**

(30) Priorität: 09.08.2012 DE 102012214152
(71) Anmelder: Deutsches Zentrum für Luft- und Raumfahrt e.V., 51147 Köln (DE)
(72) Erfinder: Bahls, Thomas, 86947 Weil (DE); Fröhlich, Florian Alexander, 82110 Germering (DE)
(74) Vertreter: von Kreisler Selting Werner

(57) **Zusammenfassung**

Verfahren zum gezielten Aufbringen eines Flüssigkeitsstrahls (10) auf und/oder in menschliche oder tierische Haus (12), mit den Schritten:
a) Erfassen der Position, Lage und Form des zu bearbeitenden Hautstücks (14), wobei diese Informationen gespeichert werden,
b) Abfahren des zu bearbeitenden Hautstücks (14) mit einer Flüssigkeitsdüse (16), die durch einen Roboterarm (18) geführt wird, wobei innerhalb der Grenzen des zu bearbeitenden Hautstücks, dessen Position, Lage und Form bekannt sind, der Flüssigkeitsstrahl (10) auf das Hautstück (14) aufgebracht wird,

wobei die Flüssigkeit (10) impulsartig in und/oder auf die Haut (12) aufgebracht wird und
an jedem Punkt des zu bearbeitenden Hautstücks (14) zu jedem Bearbeitungszeitpunkt jeweils nur ein Flüssigkeitsstrahlimpuls aufgebracht wird und der nächste Impuls an einem anderen Punkt des zu bearbeitenden Hautstücks (14) aufgebracht wird, um einen Rückstau von Flüssigkeit in der Haut (12) zu vermeiden, um so eine Tiefenwirkung des Flüssigkeitsstrahls (12) zu erreichen.

## Beschreibung

Die Erfindung betrifft ein Verfahren sowie eine Vorrichtung zum gezielten Aufbringen eines Flüssigkeitsstrahls auf und/oder in menschliche oder tierische Haut.

Das Aufbringen und Entfernen von Tätowierungen auf der Haut ist eine schwierige Aufgabe, die nur von gut ausgebildeten Personen wahrgenommen werden kann. Bislang werden die genannten Tätigkeiten manuell durchgeführt, sodass die Genauigkeit beim Erstellen einer Tätowierung oder bei seiner Entfernung von der Geschicklichkeit der durchführenden Person abhängt.

Aufgabe der Erfindung ist es ein Verfahren bereitzustellen, mit dem ein Flüssigkeitsstrahl, insbesondere Tätowierfarbe oder Wasser zum Entfernen einer Tätowierung gezielt auf und/oder in die Haut eingebracht werden kann.

Die Lösung der Aufgabe erfolgt erfindungsgemäß durch die Merkmale der Ansprüche 1 und 10.

Beim erfindungsgemäßen Verfahren wird ein Flüssigkeitsstrahl gezielt auf und/oder in die menschliche oder tierische Haut eingebracht. Das Verfahren umfasst die folgenden Schritte:
a) Zunächst wird die Position, Lage und Form des zu bearbeitenden Hautstücks erfasst und gespeichert. Dies ist notwendig, um eine genaue Kenntnis über die geometrischen Eigenschaften des zu bearbeitenden Hautstücks zu haben, sodass dieses anschließend präzise bearbeitet werden kann. Hierzu ist es möglich, zunächst die geometrischen Eigenschaften des zu bearbeitenden Körperteils an sich zu erfassen. Hierzu ist es bspw. möglich, die äußere Kontur des Körperteils zu scannen. Soll eine Tätowierung entfernt werden, ist es weiterhin möglich, in einem nächsten Verfahrensschritt z.B. unter Verwendung einer Bildbearbeitungssoftware die zu bearbeitende Hautpartie zu erkennen und festzulegen. Hierzu kann die Textur der Tätowierung automatisiert erkannt werden, die sich von der Textur der übrigen Haut unterscheidet.
b) Es erfolgt ein insbesondere repetitives Abfahren des zu bearbeitenden Hautstücks mit einer Flüssigkeitsdüse, die durch einen Roboterarm geführt wird. Innerhalb der Grenzen des zu bearbeitenden Hautstücks, dessen Position, Lage und Form bekannt sind, wird hierbei der Flüssigkeitsstrahl bevorzugt in einer oszillierenden Bewegung auf das Hautstück aufgebracht. Oszillierend bedeutet in diesem Zusammenhang, dass der Flüssigkeitsstrahl mit gleichbleibender oder sich ändernder Geschwindigkeit innerhalb der Grenzen des zu bearbeitenden Hautstücks hin und her schwenkt und somit die gesamte Fläche des zu bearbeitenden Hautstücks nach und nach bearbeitet ohne hierbei für längere Zeit auf einer Position zu verbleiben. Repetitiv bedeutet, dass jeder Punkt des zu bearbeitenden Hautstücks nicht nur einmalig sondern mehrmals vom Flüssigkeitsstrahl bearbeitet wird. Anstelle einer oszillierenden Bewegung kann das zu bearbeitende Hautstück auch in einer anderen Bewegungsform abgefahren werden. Wichtig ist lediglich, dass jeder Punkt des zu bearbeitenden Hautstücks in ausreichender Weise bearbeitet wird. Der Flüssigkeitsstrahl wird hierbei lediglich für eine vorbestimmte Höchstdauer in einen bestimmten Punkt der Haut eingebracht, sodass eine Verletzung der Haut vermieden werden kann. Weiterhin kann hierdurch ein Rückstau der Flüssigkeit in der Haut vermieden werden.

Erfindungsgemäß wird die Flüssigkeit impulsartig in und/oder auf die Haut aufgebracht. Weiterhin wird erfindungsgemäß an jedem Punkt des zu bearbeitenden Hautstücks zu jedem Bearbeitungszeitpunkt jeweils nur ein Flüssigkeitsstrahlimpuls aufgebracht und der nächste Impuls wird an einem anderen Punkt des zu bearbeitenden Hautstücks aufgebracht, um einen Rückstau von Flüssigkeit in der Haut zu vermeiden. Dieser würde sich ergeben, wenn der Flüssigkeitsstrahlimpuls zu lang ist und somit Flüssigkeit zu lange in einem Punkt der Haut eingebracht wird. Hierbei entsteht ein Rückstau oder Rückfluss von Flüssigkeit, der einen Gegendruck erzeugt, sodass der Flüssigkeitsstrahl nicht mehr tief genug in die Haut eindringen kann. Durch die genannten Verfahrensschritte ist es möglich den genannte Flüssigkeitsstau zu vermeiden und so eine Tiefenwirkung des Flüssigkeitsstrahls in der Haut zu erreichen. Eine erneute Bearbeitung desselben Punkts erfolgt erst bei einem eventuellen Wiederholungsvorgang, sofern durch das erste Aufbringen der Flüssigkeit nicht das gewünschte Ergebnis erzielt wurde.

Die auf und/oder in die Haut aufzubringende Flüssigkeit kann Tätowierfarbe sein, sodass das Verfahren ein Verfahren zum robotergestützten Aufbringen einer Tätowierung auf die Haut ist. Die aufzubringende Tätowierung muss in diesem Fall vorher in digitaler Form vorliegen und der Steuerung des Roboters zur Verfügung gestellt werden, sodass der Roboterarm derart angesteuert wird, dass die Tätowierung entsprechend der Vorlage in die Haut eingebracht wird. Durch eine entsprechende Ansteuerung des Drucks des Flüssigkeitsstrahls ist es möglich, die Tätowierfarbe in die Haut einzubringen, so wie es beim Tätowieren mit Nadeln ebenfalls geschieht. Wesentlich bei diesem Merkmal ist, dass die Flüssigkeit selber unter Druck gesetzt wird, bevor sie in die Haut eingebracht wird. Vorteilhaft am erfindungsgemäßen Verfahren ist die hohe Präzision, die durch die robotergestützte Führung der Flüssigkeitsdüse erreicht werden kann. Es ist somit möglich komplexe Tätowierungen vorzunehmen, ohne dass hierfür ein besonders geschickter Tätowierer notwendig wäre.

Alternativ kann die auf und/oder in die Haut aufzubringende Flüssigkeit Wasser oder eine andere Flüssigkeit sein, die zum Entfernen einer Tätowierung robotergestützt in die Haut eingebracht wird. Auch bei dieser Ausführungsform kann durch das robotergestützte Einbringen der Flüssigkeit eine hohe Präzision erreicht werden.

In dieser Ausführungsform ist es bevorzugt, dass die geometrische Ausgestaltung der zu entfernenden Tätowierung auf der Haut automatisch optisch erfasst und gespeichert wird und der Wasserstrahl zum Entfernen der Tätowierung ausschließlich auf Hautpartien aufgebracht wird, in denen sich Tätowierfarbe befindet. Eine Verletzung der anderen Hautpartien, die nicht bearbeitet werden müssen, kann hierdurch vermieden werden. Bei aus dem Stand der Technik bekannten Verfahren zum Entfernen von Tätowierungen ist eine solche selektive Bearbeitung von Hautgewebe nicht möglich.

Es ist bevorzugt, dass die Position, Lage und Form des zu bearbeitenden Hautstücks dynamisch, d.h. auch während des Aufbringens der Flüssigkeit in und/oder auf die Haut erfolgt, sodass z.B. eine Veränderung der Lage der Haut erfasst werden kann. Die Ansteuerung des Roboterarms wird dann entsprechend an die veränderte Position, Lage und /oder Form des zu bearbeitenden Hautstücks angepasst.

Das Erfassen der Position, Lage und Form des zu bearbeitenden Hautstücks gemäß Verfahrensschritt a) kann dadurch erfolgen, dass ein digitales Modell des zu bearbeitenden Hautstücks erstellt und gespeichert wird.

Die genannten Informationen können durch ein Abtasten des zu bearbeitenden Hautstücks durch das distale Ende des Roboterarms erfasst werden, wobei beim Berühren des Hautstücks mit dem distalen Ende des Roboterarms die jeweilige räumliche Position dieses Punkts gespeichert wird. Diese Informationen können für die genannte Modellbildung verwendet werden. Alternativ kann ein Modell des zu bearbeitenden Hautstücks auch auf andere Weise erstellt werden.

Die genannten Informationen über das zu bearbeitende Hautstück können dazu verwendet werden, die Flüssigkeitsdüse exakt in Normalrichtung, d.h. lokal senkrecht zur Oberfläche der Haut aufzusetzen. Somit kann immer ein exakt definierter Flüssigkeitsdruck in die Haut eingebracht werden.

Es ist bevorzugt, dass die Position der Flüssigkeitsdüse durch ein insbesondere optisches Tracking-System erfasst wird. Dies erfolgt im laufenden Betrieb, sodass zu jedem Zeitpunkt genau bekannt ist, an welcher Position sich die Flüssigkeitsdüse gerade befindet. Basierend auf diesen Informationen kann eine Positionsregelung der Flüssigkeitsdüse erfolgen.

Weiterhin ist es bevorzugt, dass die Kontaktkraft, mit der die Flüssigkeitsdüse auf die Haut gedrückt wird, durch einen Sensor gemessen und geregelt wird. Es ist somit möglich die Flüssigkeitsdüse immer mit einer festgelegten Maximalkraft auf die Haut zu drücken. Die Maximalkraft kann bspw. anwendungsspezifisch vorgegeben oder von der behandelnden Person einstellbar sein. Weiterhin ist es hierdurch möglich, Ungenauigkeiten im Oberflächenmodell des zu bearbeitenden Hautstücks, die zu einem zu festen Aussetzen der Düse führen würden, auszugleichen. Hierdurch können Verletzungen des Gewebes vermieden werden. Auch kann ein zu schwacher bzw. kein Kontakt mit der Oberfläche vermieden werden. In diesem Fall hätte das erfindungsgemäße Verfahren eine schlechte bzw. gar keine Wirkung.

In einer bevorzugten Ausführungsform wird die Flüssigkeit impulsartig in und/oder auf die Haut aufgebracht, wobei die Impulsdauer zwischen 10 bis 200ms liegt. Der beaufschlagte Druck liegt dabei in einem Bereich von 20 bis 200bar, bevorzugt bei 30 bis 100bar und besonders bevorzugt bei 50 bis 80bar.

Das erfindungsgemäße Verfahren eignet sich bevorzugt auch für andere kosmetische Anwendungen, in denen es wichtig ist, einen Flüssigkeitsstrahl mit hoher Genauigkeit und definiertem Druck in und/oder auf die Haut aufzubringen. Vom erfindungsgemäßen Verfahren ausgeschlossen sind medizinische Anwendungen, insbesondere chirurgische oder therapeutische Verfahren am menschlichen und/oder tierischen Körper.

Es ist ferner bevorzugt, dass nach einem ersten insbesondere repetitiven Abfahren des zu behandelnden Hautstücks gemäß dem o.g. Verfahrensschritt b) die geometrische Ausgestaltung der zu entfernenden Tätowierung erneut automatisch optisch erfasst und gespeichert wird. Anschließend wird der Roboterarm auf Basis dieser Daten erneut derart angesteuert, dass der Wasserstrahl zum Entfernen der Tätowierung ausschließlich auf Hautpartien aufgebracht wird, in denen sich noch verbliebene Tätowierfarbe befindet.

Die Erfindung betrifft ferner einen Roboter zum gezielten Aufbringen eines Flüssigkeitsstrahls auf und/oder in menschliche oder tierische Haut. Der Roboter weist einen Roboterarm auf, an dessen distalen Ende eine Flüssigkeitsdüse angebracht ist. Ferner ist eine Speichervorrichtung vorgesehen, die zum Speichern der Position, Lage und Form des zu bearbeitenden Hautstücks ausgestaltet ist. Der Roboter weist ferner eine Steuervorrichtung zum Steuern der Bewegung des Roboterarms auf, die ausgebildet ist, zum Ausgeben von Steuerkommandos, zum insbesondere repetitiven Abfahren des zu bearbeitenden Hautstücks durch den Roboterarm und zum gleichzeitigen Ansteuern der Flüssigkeitsdüse derart, dass innerhalb der Grenzen des zu bearbeitenden Hautstücks, dessen Position, Lage und Form bekannt sind, ein Aufbringen des Flüssigkeitsstrahls auf das Hautstück erfolgt.

Im Folgenden wird eine bevorzugte Ausführungsform der Erfindung anhand einer Figur erläutert.

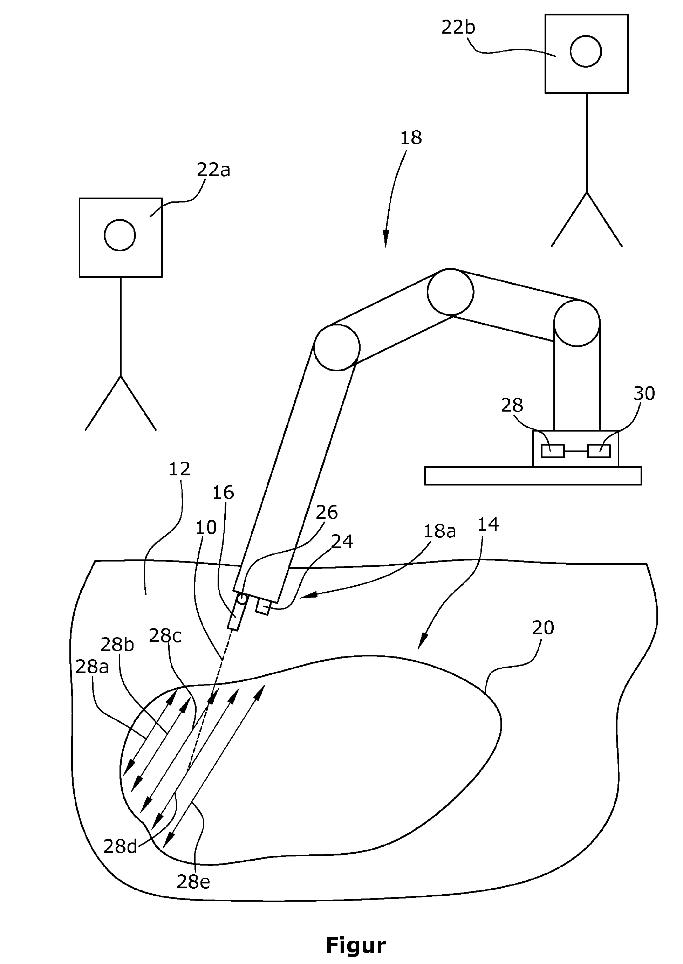

In Figur 1 ist der erfindungsgemäße Roboter 18 dargestellt, an dessen distalen Ende 18a eine Flüssigkeitsdüse 16 angebracht ist. Ferner kann an dem distalen Ende 18a eine Messvorrichtung 24 angebracht sein, mit der der zu bearbeitende Hautbereich 14 dreidimensional erfasst werden kann. Dies kann zu Modellierungs- und Planungszwecken vor dem Aufbringen der Flüssigkeit erfolgen und anschließend während des laufenden Betriebs, indem kontinuierlich in ausreichend hoher Taktrate, die Position, Lage und Form des zu bearbeitenden Hautstücks 14 erfasst wird. Hierbei ist es ausreichend die genannten Informationen in Relation zum distalen Ende 18a des Roboterarms 18 zu erfassen. Bei der Messvorrichtung 24 kann es sich bspw. um eine Stereokamera, einen PMD-Sensor und eine Kamera, einen Lichtschnittsensor und eine Kamera etc. handeln.

Der genaue Einsatzbereich des Roboterarms 18 auf der Haut 12, d.h. das zu bearbeitende Hautstück 14 kann von der behandelnden Person in einer Bildschirmdarstellung festgelegt werden. Alternativ ist es möglich, die Grenzen 20 des zu bearbeitenden Hautstücks 14 durch einen Algorithmus festzulegen, der diese Information aus den durch die Messvorrichtung 24 erfassten Bilddaten extrahiert. Dies kann bspw. im Rahmen einer Bildverarbeitung zum Erfassen der Ränder einer zu entfernenden Tätowierung erfolgen. Der automatisch festgelegte Bearbeitungsbereich kann anschließend von der behandelnden Position angepasst und freigegeben werden.

Anschließend wird der Roboterarm 18 durch die Steuerung 30, die auf den Speicher 28 zugreift, derart angesteuert, dass ein vollständiges und präzises Aufbringen von Flüssigkeit 10 in und/oder auf das zu bearbeitende Hautstück 14 erfolgt. Hierbei verbleibt der Wasserstrahl 10 immer innerhalb der Grenze 20.

Es ist bevorzugt, dass die Flüssigkeitsdüse 16 über ein Gelenk 26 mit dem distalen Ende 18a des Roboterarms 18 verbunden ist. Die Düse 16 kann somit unabhängig vom letzten Glied des Roboterarms 18 verschwenkt werden. Hierdurch kann eine vollständige Abdeckung des zu bearbeitenden Hautstücks 14 erreicht werden, ohne, dass ein großer Verfahrweg des Roboterarms 18 notwendig ist. Bspw. kann die Düse 16 mit einer hohen Geschwindigkeit sinusförmig um das Gelenk 16 verschwenkt werden, sodass der Flüssigkeitsstrahl 10 an jeder Roboterposition ein komplettes Linienstück 28 des zu bearbeitenden Hautstücks 14 überstreicht. Bspw. kann die Bearbeitung des Hautstücks 14 mit den Linienstück 28a beginnen, wonach der Roboterarm 18 ein Stück verschwenkt wird, sodass der Flüssigkeitsstrahl das Linienstück 28b bearbeitet usw. Somit ist es möglich die gesamte Oberfläche des Zielbereichs in kürzerer Zeit zu bearbeiten.

Zum Entfernen einer Tätowierung ist es bevorzugt, dass die Flüssigkeitsdüse unmittelbar auf das zu bearbeitende Hautstück 14 aufgesetzt wird. Anschließend wird der Wasserstrahl 10 bei einer kurzen Zeitspanne mit hohem Druck aktiviert. Hiernach wird die Düse wieder angehoben und zur nächsten Kontaktstelle mit der Haut 12 gefahren. Dieser Vorgang wird solange wiederholt bis das gesamte Zielgebiet 14 behandelt ist.

## Patentansprüche

1. Verfahren zum gezielten Aufbringen eines Flüssigkeitsstrahls (10) auf und/oder in menschliche oder tierische Haus (12), mit den Schritten:
a) Erfassen der Position, Lage und Form des zu bearbeitenden Hautstücks (14), wobei diese Informationen gespeichert werden,
b) Abfahren des zu bearbeitenden Hautstücks (14) mit einer Flüssigkeitsdüse (16), die durch einen Roboterarm (18) geführt wird, wobei innerhalb der Grenzen des zu bearbeitenden Hautstücks, dessen Position, Lage und Form bekannt sind, der Flüssigkeitsstrahl (10) auf das Hautstück (14) aufgebracht wird,
wobei die Flüssigkeit (10) impulsartig in und/oder auf die Haut (12) aufgebracht wird und
an jedem Punkt des zu bearbeitenden Hautstücks (14) zu jedem Bearbeitungszeitpunkt jeweils nur ein Flüssigkeitsstrahlimpuls aufgebracht wird und der nächste Impuls an einem anderen Punkt des zu bearbeitenden Hautstücks (14) aufgebracht wird, um einen Rückstau von Flüssigkeit in der Haut (12) zu vermeiden, um so eine Tiefenwirkung des Flüssigkeitsstrahls (12) zu erreichen.

2. Verfahren nach Anspruch 1, **dadurch gekennzeichnet, dass** das Erfassen der Position, Lage und Form des zu bearbeitenden Hautstücks (14) gemäß Verfahrensschritt a) dadurch erfolgt, dass ein digitales Modell des zu bearbeitenden Hautstücks (14) erstellt und gespeichert wird.

3. Verfahren nach Anspruch 1 oder 2, **dadurch gekennzeichnet, dass** das Erfassen der Position, Lage und Form des zu bearbeitenden Hautstücks (14) gemäß Verfahrensschritt a) durch ein Abtasten des zu bearbeitenden Hautstücks (14) durch das distale Ende (18a) des Roboterarms (18) erfolgt, wobei beim Berühren des Hautstücks (14) mit dem distalen Ende (18a) des Roboterarms (18) die jeweilige räumliche Position dieses Punkts gespeichert wird.

4. Verfahren nach einem der Ansprüche 1 bis 3, **dadurch gekennzeichnet, dass** die Kontaktkraft, mit der die Flüssigkeitsdüse (16) auf die Haut (12) gedrückt wird, durch einen Sensor gemessen und geregelt wird.

5. Verfahren nach einem der Ansprüche 1 bis 4, **dadurch gekennzeichnet, dass** die auf und/oder in die Haut (12) aufzubringende Flüssigkeit (10) Tätowierfarbe ist, sodass das Verfahren ein Verfahren zum robotergestützten Aufbringen einer Tätowierung auf die Haut (12) ist.

6. Verfahren nach einem der Ansprüche 1 bis 4, **dadurch gekennzeichnet, dass** die auf und/oder in die Haut (12) aufzubringende Flüssigkeit (10) Wasser oder eine andere Flüssigkeit ist, die zum Entfernen einer Tätowierung robotergestützt in die Haut (12) eingebracht wird.

7. Verfahren nach Anspruch 6, **dadurch gekennzeichnet, dass** die geometrische Ausgestaltung der zu entfernenden Tätowierung auf der Haut (12) automatisch optisch erfasst und gespeichert wird und der Roboterarm (18) auf Basis dieser Daten derart angesteuert wird, dass der Wasserstrahl (10) zum Entfernen der Tätowierung ausschließlich auf Hautpartien (14) aufgebracht wird, in denen sich Tätowierfarbe befindet.

8. Verfahren nach einem der Ansprüche 1 bis 7, **dadurch gekennzeichnet, dass** die Flüssigkeit (10) impulsartig in und/oder auf die Haut (12) aufgebracht wird, wobei die Impulsdauer des Flüssigkeitsstrahls zwischen 10 bis 200ms liegt.

9. Verfahren nach Anspruch 7, **dadurch gekennzeichnet, dass** nach einem ersten insbesondere repetitiven Abfahren des zu behandelnden Hautstücks (14) gemäß Verfahrensschritt b) die geometrische Ausgestaltung der zu entfernenden Tätowierung auf der Haut (12) gemäß Anspruch 7 erneut erfasst wird und der Wasserstrahl erneut zum Entfernen der noch verbliebenden Tätowierung ausschließlich auf Hautpartien (14) aufgebracht wird, in denen sich noch Tätowierfarbe befindet.

10. Roboter zum gezielten Aufbringen eines Flüssigkeitsstrahls (10) auf und/oder in menschliche oder tierische Haut, mit
einen Roboterarm (18), an dessen distalem Ende (18a) eine Flüssigkeitsdüse (16) angebracht ist.
einer Speichervorrichtung (24), die zum Speichern der Position, Lage und Form des zu bearbeitenden Hautstücks (14) ausgestaltet.
einer Steuervorrichtung (30) zum Steuern der Bewegungen des Roboterarms (18), die ausgebildet ist, zum Ausgeben von Steuerkommandos, zum Abfahren des zu bearbeitenden Hautstücks (14) durch den Roboterarm (18) und zum gleichzeitigen Ansteuern der Flüssigkeitsdüse (16) derart, dass innerhalb der Grenzen (20) des zu bearbeitenden Hautstücks (14), dessen Position, Lage und Form bekannt sind, ein Aufbringen des Flüssigkeitsstrahls (10) auf das Hautstück (14) erfolgt
